# EUROPÄISCHE PATENTSCHRIFT

(11) **EP 1 278 726 B1**
(45) Veröffentlichungstag und Bekanntmachung des Hinweises auf die Patenterteilung: **08.07.2009**
(21) Anmeldenummer: 01927931.4
(22) Anmeldetag: 30.04.2001
(51) Int. Cl.: C07D 201/08

(54) **VERFAHREN ZUR HERSTELLUNG CYCLISCHER LACTAME**
METHOD FOR PRODUCING CYCLIC LACTAMS
PROCEDE DE PREPARATION DE LACTAMES CYCLIQUES

(30) Priorität: 03.05.2000 DE 10021193
(43) Veröffentlichungstag der Anmeldung: 29.01.2003
(73) Patentinhaber: BASF SE, 67056 Ludwigshafen (DE)
(72) Erfinder: OHLBACH, Frank, 40219 Düsseldorf (DE); ANSMANN, Andreas, 69168 Wiesloch (DE); BASSLER, Peter, 68519 Viernheim (DE); FISCHER, Rolf-Hartmuth, 69121 Heidelberg (DE); LUYKEN, Hermann, 67069 Ludwigshafen (DE); MAIXNER, Stefan, 68723 Schwetzingen (DE); MELDER, Johann-Peter, 67459 Böhl-Iggelheim (DE)
(86) Internationale Anmeldenummer: PCT/EP2001/004841
(87) Internationale Veröffentlichungsnummer: WO 2001/083444

(56) Entgegenhaltungen:
- EP-A- 0 659 741
- WO-A-96/22974
- WO-A-98/05636
- WO-A-98/37063
- WO-A-99/28296
- WO-A-99/47500
- US-A- 5 495 016
- US-A- 5 693 793

## Beschreibung

Die vorliegende Erfindung betrifft ein Verfahren zur Herstellung cyclischer Lactame der Formel (II) 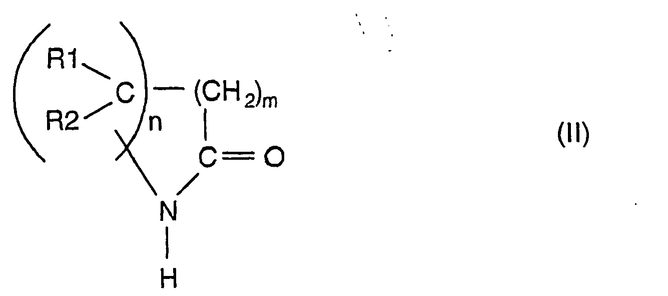 in der n und m jeweils die Werte 0, 1, 2, 3, 4, 5, 6, 7, 8 und 9 haben können und die Summe aus n + m mindestens 3, vorzugsweise mindestens 4 beträgt, R¹ und R² C₁-C₆-Alkyl-, C₅-C₇-Cycloalkyl- oder C₆-C₁₂-Arylgruppen bedeuten
durch Umsetzung einer Verbindung (I) der Formel 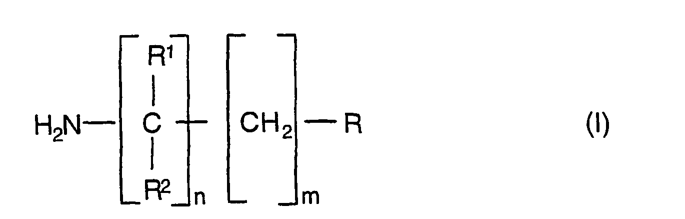 in der R¹, R², m und n die oben genannte Bedeutung besitzen und R Nitril-, Carbonsäureamid- und Carbonsäuregruppen bedeuten
mit Wasserdampf in der Gasphase, **dadurch gekennzeichnet, daß** man
a) die Verbindung (I) mit Wasserdampf in der Gasphase umsetzt, wobei man vor oder nach der Umsetzung ein organisches Verdünnungsmittel (III) zusetzt, das mit Wasser eine Mischungslücke unter bestimmten Mengen-, Druck- und Temperaturbedingungen aufweist,
   unter Erhalt einer Mischung (IV) enthaltend ein Lactam (II)
b) Mischung (IV) vor oder nach der Abtrennung von Ammoniak in Mengen-, Druck- und Temperaturbedingungen überführt, unter denen Verdünnungsmittel (III) und Wasser flüssig vorliegen und eine Mischungslücke aufweisen, unter Erhalt eines Zweiphasensystems aus einer Phase (V), die einen höheren Anteil an Verdünnungsmittel (III) als Wasser aufweist, und einer Phase (VI), die einen höheren Anteil an Wasser als an Verdünnungsmittel (III) aufweist,
c) Phase (V) von Phase (VI) abtrennt und,
d) von Phase (V) das Verdünnungsmittel (III) und gegebenenfalls Nebenprodukte ausgewählt aus der Gruppe bestehend aus Leichtsieder, Hochsieder und nicht umgesetzter Verbindung (I) abtrennt unter Erhalt eines Lactams (II).

Verfahren zur Herstellung cyclischer Lactame durch Umsetzung von omega-Aminocarbonsäurederivaten, beispielsweise die Herstellung von Caprolactam aus 6-Aminocarbonsäurenitril, mit Wasserdampf in Gegenwart von heterogenen Katalysatoren in der Gasphase sind allgemein bekannt.

So ist aus WO 96/22974, EP-A-659741, WO 99/47500 und WO99/28296 bekannt, 6-Aminocapronitril in der Gasphase mit Wasserdampf in Gegenwart von heterogenen Katalysatoren zu Caprolactam und Ammoniak umzusetzen. Als heterogene Katalysatoren werden dabei beispielsweise Aluminiumoxid, Lanthanphosphate und Zirkondioxid verwendet. In WO 96/22974 wird darauf hingewiesen, daß nicht ausgeschlossen wird, bei der Cyclisierung ein unter den Reaktionsbedingungen inertes Verdünnungsmittel, beispielsweise ein Alkan, ein Cycloalkan, einen aromatischen Kohlenwasserstoff oder einen halogenierten Kohlenwasserstoff zu verwenden und so eine Flüssigphase im Reaktionsgesmisch zu haben.

WO 98/05636 beschreibt die Aufarbeitung von Reaktionsausträgen, wie sie bei der Gasphasencyclisierung von 6-Aminocapronitril mit Wasserdampf in Gegenwart von festen Katalysatoren anfallen. Hierzu wird
a) die Hauptmenge des bei der Cyclisierung entstandenen Ammoniaks aus dem wasserhaltigen Rohcaprolactam abgetrennt und
b) dieses Rohcaprolactam entweder einer flüssig/flüssig-Extraktion unter Zusatz eines saure Gruppen enthaltenden Lösungsmittels unterworfen und/oder mit einem Kationenaustauscher behandelt.

Durch Maßnahme b) werden alle Amine extrahiert oder am Kationentauscher gebunden. Nachteilig bei dieser Aufarbeitung ist vor allem, daß in der Cyclisierung nicht umgesetztes Aminocapronitril von den Ionentauschern aufgenommen wird und bei der Regenerierung des Ionentauschers als Ammoniumsalz im Gemisch mit Ammoniumsalzen von Amin-Nebenprodukten anfällt. Es kann also nicht in die Cyclisierung zurückgeführt werden. Auch bei der Extraktion des Aminocapronitrils mit Säuregruppen enthaltenden Extraktionsmitteln entstehen Ammoniumsalze, die die gleichen Probleme mit sich bringen.

Die Aufgabe der vorliegenden Anmeldung bestand daher darin, ein Verfahren bereitzustellen, das die Herstellung von cyclischen Lactamen (II) aus Verbindungen (I) auf technisch einfache und wirtschaftliche Weise ermöglicht, bei hohen Umsätzen an Verbindung (I) zu hohen Lactam-Ausbeuten führt und zudem Ausbeuteverluste bei der Aufarbeitung minimiert.

Demgemäß wurde das eingangs definierte Verfahren gefunden.

Als Verbindung (I) kommen Aminocarbonsäuren und ihre Derivate vorzugsweise solche der allgemeinen Formel I 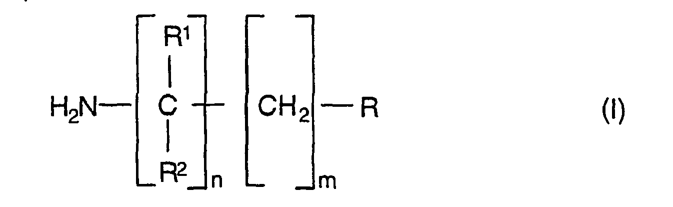 in Betracht, wobei R Carbonsäure-, Nitril- und/oder Carbonsäureamidgruppen bedeutet und n und m jeweils die Werte 0, 1, 2, 3, 4, 5, 6, 7, 8 und 9 haben können und die Summe aus n + m mindestens 3, vorzugsweise mindestens 4 beträgt.

R¹ und R² können prinzipiell Substituenten jeglicher Art sein, wobei lediglich sichergestellt sein sollte, dass die gewünschte Cyclisierungsreaktion durch die Substituenten nicht beeinflusst wird. Vorzugsweise sind R ¹ und R² unabhängig voneinander C₁-C₆-Alkyl- oder C₅-C₇-Cycloalkylgruppen oder C₆-C₁₂-Arylgruppen.

Besonders bevorzugte Ausgangsverbindungen sind Aminocarbonsäurenitrile, vorzugsweise der allgemeinen Formel

H₂N-(CH₂)ₘ-C≡ N

wobei m einen Wert von 3, 4, 5 oder 6, insbesondere 5 aufweist. Für m = 5 ergibt sich als Ausgangsverbindung 6-Aminocapronsäurenitril.

Als Verbindung (I) kann eine einzelne Verbindung (I) oder ein Gemisch verschiedener Verbindungen (I) eingesetzt werden. Vorzugsweise setzt man als Verbindung (I) eine einzelne Verbindung ein.

Omega-Aminocarbonsäurenitrile sind beispielsweise durch partielle Hydrierung von alpha, omega-Dinitrilen in der Gas- oder Flüssigphase, z. B. nach WO 96/20166, WO 96/20916 oder WO 96/20165, zugänglich.

Omega-Aminocarbonsäuren sind beispielsweise durch aminierende Hydrierung von omega-Formylcarbonsäuren oder durch Hydrolyse von omega-Aminocarbonsäureestern oder omega-Aminocarbonsäurenitrilen zugänglich.

Omega-Aminocarbonsäureamide sind beispielsweise durch Umsetzung von omega-Aminocarbonsäuren und ihren Estern mit Ammoniak oder omega-Aminocarbonsäurenitrilen mit Wasser zugänglich.

In dem erfindungsgemäßen Verfahren erhält man in Abhängigkeit von Verbindung (I) die entsprechenden cyclischen Lactame der Formel (II) 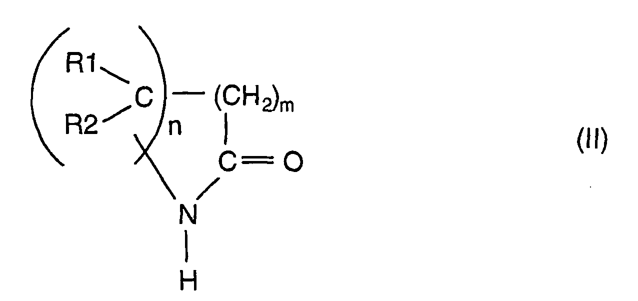 wobei, n, m , R¹ und R² die vorstehend genannte Bedeutung haben. Besonders bevorzugte Lactame sind solche, in denen n = 0 ist und m einen Wert von 3, 4, 5 oder 6 hat. Für m=5 ergibt sich als Produkt Caprolactam.

In dem erfindungsgemäßen Verfahren setzt man in Schritt a) die vorstehend beschriebenen Verbindung (I) mit Wasserdampf in der Gasphase, vorteilhaft in Gegenwart eines heterogenen Katalysators, gegebenenfalls in Gegenwart eines organischen Verdünnungsmittel (III) zu einer Mischung (IV) enthaltend ein Lactam (II) um.

Als heterogene Katalysatoren sind alle für die Gasphasencyclisierung von Verbindung (I) zu Lactam (II) beschriebenen Katalysatoren, wie Titandioxid, amorph, als Anatas oder Rutil, Aluminiumoxid, Lanthanphosphate, Hafniumoxid, Zirkondioxid oder deren Gemische, vorzugsweise Titandioxid, Aluminiumoxid, Lanthanphosphate, Zirkondioxid oder deren Gemische, geeignet.

Verdünnungsmittel (III) kann vor der Cyclisierung zugesetzt werden. In diesem Fall ist Verdünnungsmittel (III) während der Cyclisierung von Verbindung (I) im gasförmigen Reaktionsgemisch anwesend, vorzugsweise in Mengen von 0,1 bis 20 g Verdünnungsmittel (III) pro Gramm Verbindung (I).

Vorzugsweise kommt die Zugabe von Verdünnungsmittel (III) zu dem Cyclisierungsaustrag in Betracht, beispielsweise durch Quenchen des Cyclisierungsaustrags.

Weiterhin kann das Verdünnungsmittel (III) nach der Abtrennung von Ammoniak aus Mischung (IV) zugesetzt werden.

Als Verdünnungsmittel (III) können solche organischen Verbindungen eingesetzt werden, die mit Wasser eine Mischungslücke unter bestimmten Mengen-, Druck- und Temperaturbedingungen, insbesondere unterhalb der Reaktionstemperatur bei der Cyclisierung, aufweisen.

Als Verdünnungsmittel (III) kommen C₄- bis C₉-Alkanole, wie n-Butanol, i-Butanol, n-Pentanol, vorzugsweise aliphatische Kohlenwasserstoffe, wie n-Hexan, cycloaliphatische Kohlenwasserstoffe, wie Cyclopentan oder Cyclohexan, insbesondere aromatische Kohlenwasserstoffe, wie Benzol, Toluol, o-Xylol, m-Xylol, p-Xylol, Ethylbenzol, i-Propylbenzol, Di-i-propylbenzol sowie Gemische solcher Verbindungen, beispielsweise Petrolether, in Betracht. Die Kohlenwasserstoffe können funktionelle Gruppen, wie Halogene, beispielsweise Chlor, tragen, wie in Chlorbenzol.

Bei der Umsetzung gemäß Schritt a) kann auch Ammoniak anwesend sein.

Die Umsetzung kann vorteilhaft in der Gasphase bei Temperaturen von im allgemeinen 200 bis 450°C, vorzugsweise 250 bis 400°C durchgeführt werden. Der Druck sollte im allgemeinen im Bereich von 0,01 bis 20 bar, vorzugsweise von 0,1 bis 5 bar liegen. Dabei sollte das Reaktionsgemisch zum überwiegenden Teil gasförmig vorliegen. Die Verweilzeiten liegen im allgemeinen im Bereich von 0,1 bis 100, vorzugsweise 1 bis 50 Sekunden.

Bei der Cyclisierung sollte pro Mol Verbindung (I) im allgemeinen mindestens 0,5 mol, vorzugsweise mindestens 1 bis 50 mol Wasser eingesetzt werden.

Die Umsetzung kann in Gegenwart eines inerten Gases, wie Stickstoff oder Edelgas oder Gemische solcher Gase, durchgeführt werden.

Bei der Umsetzung gemäß Schritt a) wird eine Mischung (IV) erhalten, die ein Lactam (II) enthält.

Die Abtrennung von Ammoniak gemäß Schritt b) kann, sofern Mischung (IV) Ammoniak enthält, nach, vorzugsweise vor der Phasentrennung aus dem Gemisch (IV) erfolgen, vorzugsweise durch Destillation, unter Erhalt einer Ammoniak-freien oder -armen Mischung (IX). Eine Abtrennung des Ammoniaks kann auch nach der Phasentrennung erfolgen, vorzugsweise durch Destillation, aus Phase (V) und/oder Phase (VI).

Mischung (IV) kann Ammoniak beispielsweise enthalten, wenn bei der Umsetzung gemäß Schritt a) Ammoniak entstanden ist und/oder wenn der Reaktionsmischung bei der Umsetzung gemäß Schritt a) Ammoniak zugesetzt worden ist. Bei der Umsetzung gemäß Schritt a) kann beispielsweise Ammoniak entstehen, wenn R eine Nitril- oder Carbonsäureamidgruppe ist.

Die Abtrennung kann vorteilhaft destillativ erfolgen, insbesondere bei Sumpftemperaturen von 60 bis 220°C und Drücken von 1 bis 30 bar.

Enthält Mischung (IV) keinen Ammoniak, wobei hierunter auch solche geringen Spuren an Ammoniak verstanden werden, die keine Beeinträchtigung der folgenden Verfahrensschritte darstellen, so sind Mischung (IV) und Mischung (IX) identisch.

Gemäß Schritt b) wird erfindungsgemäß Mischung (IX) in Mengen-, Druck- und Temperaturbedingungen überführt, unter denen Verdünnungsmittel (III) und Wasser flüssig vorliegen und eine Mischungslücke aufweisen, unter Erhalt eines Zweiphasensystems aus einer Phase (V) und einer Phase (VI).

Bevorzugt sind solche Mengen-, Druck- und Temperaturbedingungen, unter denen die Bestandteile der Mischung (VII) in den Phasen (V) und (VI) vollständig flüssig vorliegen, also keine Feststoffe ausfallen.

Wurde Schritt a) in einer homogenen Flüssigphase durchgeführt, so kann im allgemeinen eine Auftrennung der Mischung (VII) in die beiden Phasen (V) und (VI) durch Wahl einer geeigneten Temperatur erreicht werden. Als weitere Möglichkeit kommt die Wahl geeigneter Mengenverhältnisse in Betracht wie der Zusatz von Verdünnungsmittel (III), vorzugsweise von Wasser.

Anschließend werden erfindungsgemäß Phase (V) und Phase (VI) gemäß Schritt c) getrennt.

Die Phasentrennung kann in an sich bekannter Weise in für solche Zwecke beschriebenen Apparaturen erfolgen, wie sie beispielsweise aus: Ullmann's Encyclopedia of Industrial Chemistry, Vol. B3, 5. Ed., VCH Verlagsgesellschaft, Weinheim, 1988, Seite 6-14 bis 6-22 bekannt sind.

Die für die Phasentrennung optimalen Apparaturen und Verfahrensbedingungen lassen sich dabei leicht durch einige einfache Vorversuche ermitteln.

Gemäß Schritt d) werden erfindungsgemäß von Phase (V) das Verdünnungsmittel (III) und gegebenenfalls Ammoniak und gegebenenfalls Nebenprodukte ausgewählt aus der Gruppe bestehend aus Leichtsieder (VIII), Hochsieder (VII) und nicht umgesetzter Verbindung (I) abgetrennt unter Erhalt eines Lactams (II).

Unter Leichtsieder (VIII) werden dabei im Sinne der vorliegenden Erfindung Verbindungen mit einem Siedepunkt unterhalb dessen von Lactam (II) verstanden, unter Hochsieder (VII) solche Verbindungen mit einem Siedepunkt oberhalb dessen von Lactam (II).

Diese Aufarbeitung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Vorzugsweise kommt zunächst die Abtrennung von gegebenenfalls noch vorhandenem Ammoniak und Verdünnungsmittel (III) von Phase (V) in Betracht. Von dem Lactam (II) können dann die Hochsieder (VII), Leichtsieder (VIII) und gegebenenfalls nicht umgesetzte Verbindung (I) einzeln oder gemeinsam abgetrennt werden.

Vorteilhaft kann das in Schritt d) anfallende Verdünnungsmittel (III) teilweise oder vollständig in Schritt a) zurückgeführt werden.

Vorteilhaft können in Schritt d) gegebenenfalls anfallende Hochsieder (VIII) und/oder Leichtsieder (VII) teilweise oder vollständig in Schritt a) zurückgeführt werden.

Vorteilhaft kann in Schritt d) gegebenenfalls anfallende, nicht umgesetzte Verbindung (I) teilweise oder vollständig in Schritt a) zurückgeführt werden.

Die in Schritt c) erhaltene Phase (VI) kann vorteilhaft in Schritt a) zurückgeführt werden.

Man kann vorzugsweise aus der Phase (VI) unter Erhalt einer Mischung (X) das Lactam (II) teilweise oder vollständig abtrennen und gegebenenfalls aus dem so erhaltenen Lactam (II) Leichtsieder (VIII) und/oder Hochsieder (VII) abtrennen.

Diese Aufarbeitung des Lactams (II) kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Von dem Lactam (II) können die Hochsieder (VII) und/oder Leichtsieder (VIII) einzeln oder gemeinsam abgetrennt werden.

Vorteilhaft können die Hochsieder (VII) und/oder Leichtsieder (VIII) teilweise oder vollständig in Schritt a) zurückgeführt werden.

Besonders bevorzugt kommt dabei eine Rückspaltung der Hochsieder (VII) vor der Rückführung in Schritt a) in Betracht, wie sie beispielsweise beschrieben ist in EP-A-793650, EP-A-793651, EP-A-794643 oder EP-A-912508.

Das aus Phase (VI) erhaltene Lactam (II) kann auch vor der Aufarbeitung mit dem aus Schritt d) erhaltenen rohen Lactam (II) vereinigt und gemeinsam aufgearbeitet werden.

Vorteilhaft kann man Phase (X) in Schritt a) zurückführen.

Man kann aus Phase (VI) Lactam (II) durch Extraktion mit einem flüssigen Extraktionsmittel (XI) teilweise oder vollständig abtrennen unter Erhalt einer Mischung (XII), die ein Extraktionsmittel (XI) und ein Lactam (II) enthält.

Als Extraktionsmittel (XI) kommen C₄- bis C₉-Alkanole, wie n-Butanol, i-Butanol, n-Pentanol, vorzugsweise aliphatische Kohlenwasserstoffe, wie n-Hexan, cycloaliphatische Kohlenwasserstoffe, wie Cyclopentan oder Cyclohexan, insbesondere aromatische Kohlenwasserstoffe, wie Benzol, Toluol, o-Xylol, m-Xylol, p-Xylol, Ethylbenzol, i-Propylbenzol, Di-i-propylbenzol sowie Gemische solcher Verbindungen, beispielsweise Petrolether, in Betracht. Die Kohlenwasserstoffe können funktionelle Gruppen, wie Halogene, beispielsweise Chlor, tragen, wie in Chlorbenzol.

Insbesondere weisen Extraktionsmittel (XI) und Verdünnungsmittel (III) die gleiche oder eine ähnliche Zusammensetzung auf.
So kann man vorteilhaft als Extraktionsmittel (XI) aus Schritt d) abgetrenntes Verdünnungsmittel (III) einsetzen.

Die bei der Extraktion verbleibende wäßrige Phase (X) kann vorteilhaft in Schritt a) zurückgeführt werden.

Vorteilhaft kann von Mischung (XII) das Extraktionsmittel (XI) und gegebenenfalls Leichtsieder (VIII), Hochsieder (VII) und/oder nicht umgesetzter Verbindung (I) abgetrennt werden unter Erhalt eines Lactams (II).

Diese Aufarbeitung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Vorzugsweise kommt zunächst die Abtrennung von Extraktionsmittel (XI) von Mischung (XII) in Betracht. Von dem Lactam (II) können dann die Hochsieder, Leichtsieder und gegebenenfalls nicht umgesetzte Verbindung (I) einzeln oder gemeinsam abgetrennt werden.

Vorteilhaft kann das bei der Aufarbeitung anfallende Exatraktionsmittel (XI) teilweise oder vollständig in Schritt a) zurückgeführt werden.

Vorteilhaft können bei der Aufarbeitung gegebenenfalls anfallende Hochsieder (VII) und/oder Leichtsieder (VIII) teilweise oder vollständig in Schritt a) zurückgeführt werden.

Vorteilhaft kann bei der Aufarbeitung gegebenenfalls anfallende, nicht umgesetzte Verbindung (I) teilweise oder vollständig in Schritt a) zurückgeführt werden.

Man kann vorteilhaft Mischung (XII) und Phase (V) gemeinsam in Schritt d) des erfindungsgemäßen Verfahrens einsetzen. Dabei können Mischung (XII) und Phase (V) vor oder während Schritt d) vereinigt werden.

Die bei dem erfindungsgemäßen Verfahren erhaltenen Lactame können in an sich bekannter Weise bei der Herstellung von technisch wichtigen Polymeren, wie Polyamiden, eingesetzt werden.

## Patentansprüche

1. Verfahren zur Herstellung cyclischer Lactame der Formel (II) 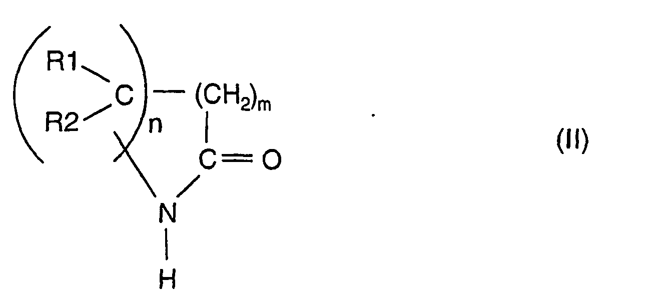 in der n und m jeweils die Werte 0, 1, 2, 3, 4, 5, 6, 7, 8 und 9 haben können und die Summe aus n + m mindestens 3, vorzugsweise mindestens 4 beträgt, R¹ und R² C₁-C₆-Alkyl-, C₅-C-₇-Cycloalkyl- oder C₆-C₁₂-Arylgruppen bedeuten
durch Umsetzung einer Verbindung (I) der Formel 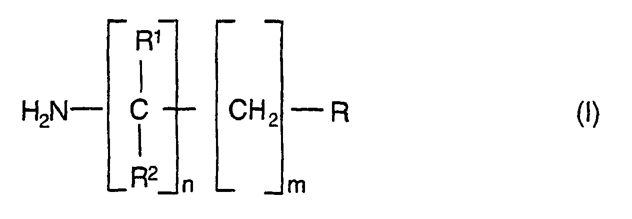 in der R¹, R², m und n die oben genannte Bedeutung besitzen und R Nitril-, Carbonsäureamid- und Carbonsäuregruppen bedeuten
mit Wasserdampf in der Gasphase, **dadurch gekennzeichnet, daß** man
a) die Verbindung (I) mit Wasserdampf in der Gasphase umsetzt, wobei man vor oder nach der Umsetzung ein organisches Verdünnungsmittel (III) zusetzt, das mit Wasser eine Mischungslücke unter bestimmten Mengen-, Druck- und Temperaturbedingungen aufweist,
unter Erhalt einer Mischung (IV) enthaltend ein Lactam (II)
b) Mischung (IV) vor oder nach der Abtrennung von Ammoniak in Mengen-, Druck- und Temperaturbedingungen überführt, unter denen Verdünnungsmittel (III) und Wasser flüssig vorliegen und eine Mischungslücke aufweisen, unter Erhalt eines Zweiphasensystems aus einer Phase (V), die einen höheren Anteil an Verdünnungsmittel (III) als Wasser aufweist, und einer Phase (VI), die einen höheren Anteil an Wasser als an Verdünnungsmittel (III) aufweist,
c) Phase (V) von Phase (VI) abtrennt und,
d) von Phase (V) das Verdünnungsmittel (III) und gegebenenfalls Nebenprodukte ausgewählt aus der Gruppe bestehend aus Leichtsieder, Hochsieder und nicht umgesetzter Verbindung (I) abtrennt unter Erhalt eines Lactams (II).

2. Verfahren nach Anspruch 1, wobei man als Verbindung (I) ein Aminocarbonsäurenitril einsetzt.

3. Verfahren nach Anspruch 1 oder 2, wobei man als Verbindung (I) ein Aminocarbonsäurenitril der Formel
H₂N-(CH₂)ₘ-C≡N
mit m 3, 4, 5 oder 6 ist, einsetzt.

4. Verfahren nach den Ansprüchen 1 bis 3, wobei man als Verbindung (I) 6-Aminocarbonsäurenitril einsetzt.

5. Verfahren nach den Ansprüchen 1 bis 4, wobei man Schritt a) in Gegenwart eines heterogenen Katalysators durchführt.

6. Verfahren nach Anspruch 5, wobei man als heterogenen Katalysator Titandioxid, Aluminiumoxid, Lanthanphosphate oder Zirkondioxid einsetzt.

7. Verfahren nach den Ansprüchen 1 bis 6, wobei man die Umsetzung in Schritt a) bei einer Temperatur im Bereich von 200 bis 450 °C durchführt.

8. Verfahren nach den Ansprüchen 1 bis 7, wobei die Summe der Konzentration von Verbindung (I) und Verbindung (II) bezogen auf Gemisch (IV) weniger als 20 Gew.-% beträgt.

9. Verfahren nach den Ansprüchen 1 bis 8, wobei man als Verdünnungsmittel (III) einen aliphatischen, cycloaliphatischen oder aromatischen Kohlenwasserstoff einsetzt.

10. Verfahren nach den Ansprüchen 1 bis 9, wobei man als Verdünnungsmittel (III) Ethylbenzol, Benzol, Toluol, o-Xylol, m-Xylol oder p-Xylol einsetzt.

11. Verfahren nach den Ansprüchen 1 bis 10, wobei man die Abtrennung von Ammoniak aus Mischung (IV) vor der Abtrennung der Phase (V) gemäß Schritt c) durchführt.

12. Verfahren nach den Ansprüchen 1 bis 11, wobei man die Umsetzung in Schritt a) in einer homogenen flüssigen Phase durchführt.

13. Verfahren nach den Ansprüchen 1 bis 12, wobei man die in Schritt c) abgetrennte Phase (VI) ganz oder teilweise in Schritt a) zurückführt.

14. Verfahren nach den Ansprüchen 1 bis 13, wobei man aus der in Schritt c) abgetrennten Phase (VI) unter Erhalt einer Phase (X), die weniger Lactam (II) als Phase (VI) enthält, das Lactam (II) teilweise oder vollständig abtrennt und gegebenenfalls aus dem so erhaltenen Lactam (II) Nebenprodukte ausgewählt aus der Gruppe bestehend aus Leichtsieder (VIII) und Hochsieder (VII) abtrennt.

15. Verfahren nach den Ansprüchen 1 bis 14, wobei man aus der Phase (VI) Lactam (II) durch Extraktion mit einem Extraktionsmittel (XI) teilweise oder vollständig abtrennt unter Erhalt einer Extraktionsmittel (XI) und Lactam (II) enthaltenden Mischung (XII) und einer Phase (X), die weniger Lactam (II) als Phase (VI) enthält.

16. Verfahren nach Anspruch 14 oder 15, wobei man Phase (X) ganz oder teilweise in Schritt a) zurückführt.

17. Verfahren nach den Ansprüchen 5 bis 16, wobei man von Mischung (XII) das Extraktionsmittel (XI) und gegebenenfalls Nebenprodukte ausgewählt aus der Gruppe bestehend aus Leichtsieder (VIII) und Hochsieder (VII) abtrennt unter Erhalt eines Lactams (II).

18. Verfahren nach den Ansprüchen 5 bis 17, wobei man Mischung (XII) und Phase (V) gemeinsam in Schritt d) einsetzt.

19. Verfahren nach den Ansprüchen 5 bis 18, wobei Extraktionsmittel (XI) und Verdünnungsmittel (III) aus den gleichen Komponenten bestehen oder die gleiche Zusammensetzung aufweisen.

20. Verfahren nach den Ansprüchen 13 bis 19, wobei man Lactam (II) nur aus dem nicht in die Cyclisierungsstufe zurückgeführten Teilstrom von Phase (VI) abtrennt und den restlichen Teil von Phase (VI) ohne Gewinnung von Lactam (II) ganz oder teilweise in Schritt a) zurückführt.

21. Verfahren nach den Ansprüchen 1 bis 20, wobei man abgetrennte, nicht umgesetzte Verbindung (I) aus Schritt d) teilweise oder vollständig in Schritt a) zurückführt.

22. Verfahren nach den Ansprüchen 1 bis 21, wobei man in Schritt d) abgetrennte Hochsieder (VII) in Schritt a) ganz oder teilweise zurückführt.

23. Verfahren nach Anspruch 22, wobei die in Schritt d) abgetrennten Hochsieder (VII) mindestens 20 Gew.-% Lactam (II) enthalten.

24. Verfahren nach den Ansprüchen 1 bis 23, wobei man in Schritt d) abgetrenntes Verdünnungsmittel (III) teilweise oder vollständig in Schritt a) zurückführt.

25. Verfahren nach den Ansprüchen 1 bis 24, wobei man in Schritt d) abgetrennte Leichtsieder (VIII) teilweise oder vollständig in Schritt a) zurückführt.

26. Verfahren nach den Ansprüchen 15 bis 25, wobei man als Extraktionsmittel (XI) aus Schritt d) abgetrenntes Verdünnungsmittel (III) einsetzt.

27. Verfahren nach den Ansprüchen 1 bis 26, wobei man aus Phase (V) oder Phase (XII) Lactam (II) ganz oder teilweise mit Wasser extrahiert unter Erhalt einer an Lactam (II) abgereichteren Phase (XIII) und einer an Lactam (II) angereichteren Phase (XIV) und Phase (XIII) ganz oder teilweise in Schritt a) zurückführt.

28. Verfahren nach den Ansprüchen 1 bis 27, wobei der in Schritt a) zurückgeführte Hochsiederstrom (VII) mit dem zurückgeführten Strom (VI) oder (X) vor Schritt a) vereinigt wird.

## Claims

1. A process for the preparation of cyclic lactams of formula (II): 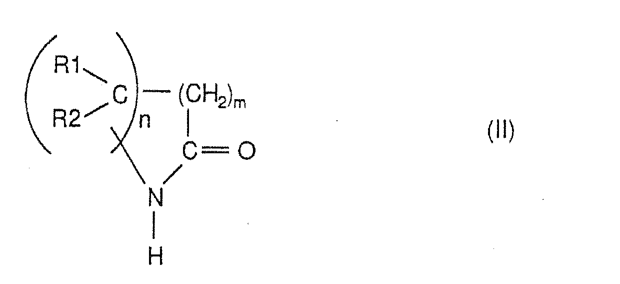 in which n and m can each have the values 0, 1, 2, 3, 4, 5, 6, 7, 8 and 9 and the sum of n + m is at least 3, preferably at least 4, and R¹ and R² are C₁-C₆-alkyl, C₅-C₇-cycloalkyl or C₆-C₁₂-aryl groups,
by reacting a compound (I) of the formula 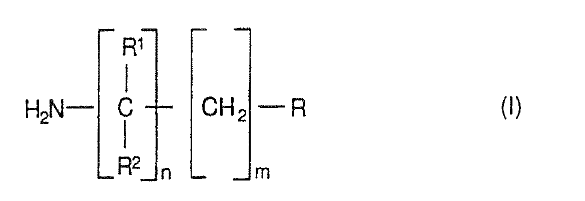 in which R¹, R², m and n are as defined above and R represents nitrile, carboxamide and carboxylic acid groups, with water vapor in the gas phase, wherein
a) the compound (I) is reacted with water vapor in the gas phase, an organic diluent (III) being added before or after the reaction, the diluent (III) exhibiting a miscibility gap with water under specific quantity, pressure and temperature conditions, to give a mixture (IV) comprising a lactam (II),
b) the mixture (IV) is converted, before or after the separation of ammonia, under quantity, pressure and temperature conditions such that the diluent (III) and water are liquid and exhibit a miscibility gap, to give a two-phase system consisting of a phase (V) comprising a higher proportion of diluent (III) than water, and a phase (VI) comprising a higher proportion of water than diluent (III),
c) the phase (V) is separated from the phase (VI), and
d) the diluent (III) and optionally by-products selected from the group consisting of low-boilers, high-boilers and unreacted compound (I) are separated from the phase (V) to give a lactam (II).

2. The process according to claim 1 wherein the compound (I) used is an aminocarboxylic acid nitrile.

3. The process according to claim 1 or 2 wherein the compound (I) used is an aminocarboxylic acid nitrile of the formula
H₂N-(CH₂)ₘ-C≡N
in which m is 3, 4, 5 or 6.

4. The process according to any of claims 1 to 3 wherein the compound (I) used is 6-aminocaproic acid nitrile.

5. The process according to any of claims 1 to 4 wherein step a) is carried out in the presence of a heterogeneous catalyst.

6. The process according to claim 5 wherein the heterogeneous catalyst used is titanium dioxide, aluminum oxide, lanthanum phosphates or zirconium dioxide.

7. The process according to any of claims 1 to 6 wherein the reaction of step a) is carried out at a temperature ranging from 200 to 450°C.

8. The process according to any of claims 1 to 7 wherein the sum of the concentration of compound (I) and compound (II) is less than 20% by weight, based on the mixture (IV).

9. The process according to any of claims 1 to 8 wherein the diluent (III) used is an aliphatic, cycloaliphatic or aromatic hydrocarbon.

10. The process according to any of claims 1 to 9 wherein the diluent (III) used is ethylbenzene, benzene, toluene, o-xylene, m-xylene or p-xylene.

11. The process according to any of claims 1 to 10 wherein the separation of ammonia from the mixture (IV) is effected before the separation of the phase (V) in step c).

12. The process according to any of claims 1 to 11 wherein the reaction of step a) is carried out in a homogeneous liquid phase.

13. The process according to any of claims 1 to 12 wherein all or part of the phase (VI) separated off in step c) is recycled into step a).

14. The process according to any of claims 1 to 13 wherein all or part of the lactam (II) is separated from the phase (VI) separated off in step c) to give a phase (X) comprising less lactam (II) than the phase (VI), and by-products selected from the group consisting of low-boilers (VIII) and high-boilers (VII) are if appropriate separated from the resulting lactam (II).

15. The process according to any of claims 1 to 14 wherein all or part of the lactam (II) is separated from the phase (VI) by extraction with an extractant (XI) to give a mixture (XII) comprising extractant (XI) and lactam (II), and a phase (X) comprising less lactam (II) than the phase (VI).

16. The process according to claim 14 or 15 wherein all or part of the phase (X) is recycled into step a).

17. The process according to any of claims 5 to 16 wherein the extractant (XI) and optionally by-products selected from the group consisting of low-boilers (VIII) and high-boilers (VII) are separated from the mixture (XII) to give a lactam (II).

18. The process according to any of claims 5 to 17 wherein the mixture (XII) and the phase (V) are used together in step d).

19. The process according to any of claims 5 to 18 wherein the extractant (XI) and the diluent (III) consist of the same components or have the same composition.

20. The process according to any of claims 13 to 19 wherein the lactam (II) is separated only from the partial stream of phase (VI) which is not returned to the cyclization step, and all or part of the remainder of phase (VI) is recycled into step a) without recovering the lactam (II).

21. The process according to any of claims 1 to 20 wherein all or part of the unreacted compound (I) separated off in step d) is recycled into step a).

22. The process according to any of claims 1 to 21 wherein all or part of the high-boilers (VII) separated off in step d) is recycled into step a).

23. The process according to claim 22 wherein the high-boilers (VII) separated off in step d) comprise at least 20% by weight of lactam (II).

24. The process according to any of claims 1 to 23 wherein all or part of the diluent (III) separated off in step d) is recycled into step a).

25. The process according to any of claims 1 to 24 wherein all or part of the low-boilers (VIII) separated off in step d) is recycled into step a).

26. The process according to any of claims 15 to 25 wherein the diluent (III) separated off in step d) is used as the extractant (XI).

27. The process according to any of claims 1 to 26 wherein all or part of the lactam (II) is extracted from the phase (V) or the phase (XII) with water to give a phase (XIII) impoverished in lactam (II) and a phase (XIV) enriched in lactam (II), and all or part of the phase (XIII) is recycled into step a).

28. The process according to any of claims 1 to 27 wherein the high-boilers stream (VII) recycled into step a) is combined with the recycled stream (VI) or (X) before step a).

## Revendications

1. Procédé de préparation de lactames cycliques de la formule (II) 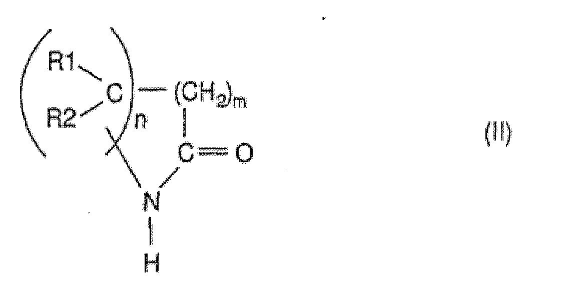 dans laquelle n et m peuvent avoir chacun les valeurs de 0, 1, 2, 3, 4, 5, 6, 7, 8 et 9 et la somme de n + m est d'au moins 3, de préférence d'au moins 4, R¹ et R² représentent des groupes alkyle en C₁-C₆, cycloalkyle en C₅-C₇ ou aryle en C₆-C₁₂,
par réaction d'un composé (I) de la formule 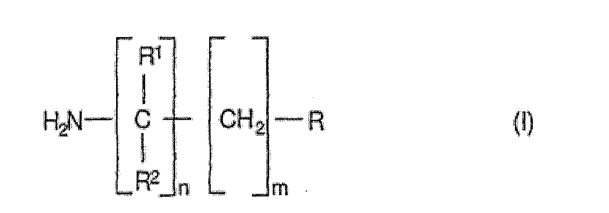 dans laquelle R¹, R², m et n présentent la signification donnée ci-dessus et R représente des groupes nitrile, amide d'acide carboxylique et acide carboxylique,
avec de la vapeur d'eau dans la phase gazeuse, **caractérisé en ce que**
a) on fait réagir le composé (I) avec de la vapeur d'eau dans la phase gazeuse, un diluant organique (III) étant ajouté avant ou après la réaction, le diluant (III) présentant avec l'eau une lacune de miscibilité dans certaines conditions de quantités, de pression et de température,
pour former un mélange (IV) contenant un lactame (II)
b) on fait passer du mélange (IV), avant ou l'isolement d'ammoniac, dans des conditions de quantités, de pression et de température dans lesquelles le diluant (III) et l'eau se trouvent à l'état liquide et présentent une lacune de miscibilité, avec obtention d'un système à deux phases à base d'une phase (V) qui présente une fraction de diluant (III) supérieure à l'eau, et d'une phase (VI), qui présente une fraction d'eau supérieure au diluant (III),
c) on isole la phase (V) de la phase (VI), et
d) à partir de la phase (V), on isole le diluant (III) et éventuellement des produits secondaires choisis parmi le groupe constitué des substances à faible point d'ébullition, des substances à haut point d'ébullition et du composé (I) qui n'a pas réagi, avec obtention d'un lactame (II).

2. Procédé suivant la revendication 1, dans lequel, comme composé (I), on met en oeuvre un nitrile d'acide aminocarboxylique.

3. Procédé suivant la revendication 1 ou 2, dans lequel, comme composé (I), on met en oeuvre un nitrile d'acide aminocarboxylique de la formule
H₂N-(CH₂)ₘ-C≡N
où m vaut 3, 4, 5 ou 6.

4. Procédé suivant les revendications 1 à 3, dans lequel, comme composé (I), on met en oeuvre du nitrile d'acide 6-aminocaproique.

5. Procédé suivant les revendications 1 à 4, dans lequel on effectue l'étape a) en présence d'un catalyseur hétérogène.

6. Procédé suivant la revendication 5, dans lequel, comme catalyseur hétérogène, on met en oeuvre du dioxyde de titane, de l'oxyde d'aluminium, des phosphates de lanthane ou du dioxyde de zirconium.

7. Procédé suivant les revendications 1 à 6, dans lequel on effectue la réaction dans l'étape a) à une température de l'ordre de 200 à 450°C.

8. Procédé suivant les revendications 1 à 7, dans lequel la somme de la concentration du composé (I) et du composé (II) est inférieure à 20 % en poids, par rapport au mélange (IV).

9. Procédé suivant les revendications 1 à 8, dans lequel, comme diluant (III), on met en oeuvre un hydrocarbure aliphatique, cycloaliphatique ou aromatique.

10. Procédé suivant les revendications 1 à 9, dans lequel, comme diluant (III), on met en oeuvre de l'éthylbenzène, du benzène, du toluène, du o-xylène, du m-xylène ou du p-xylène.

11. Procédé suivant les revendications 1 à 10, dans lequel on effectue l'isolement d'ammoniac à partir du mélange (IV) avant l'isolement de la phase (V) selon l'étape c).

12. Procédé suivant les revendications 1 à 11, dans lequel on effectue la réaction dans l'étape a) dans une phase liquide homogène.

13. Procédé suivant les revendications 1 à 12, dans lequel on recycle totalement ou partiellement dans l'étape a) la phase (VI) isolée dans l'étape c).

14. Procédé suivant les revendications 1 à 13, dans lequel on isole partiellement ou totalement le lactame (II) à partir de la phase (VI) isolée dans l'étape c), avec obtention d'une phase (X) qui contient moins de lactame (II) que la phase (VI), et éventuellement on isole à partir du lactame (II) ainsi obtenu des produits secondaires choisis parmi le groupe constitué des substances à faible point d'ébullition (VIII) et des substances à haut point d'ébullition (VII).

15. Procédé suivant les revendications 1 à 14, dans lequel, à partir de la phase (VI), on isole partiellement ou totalement du lactame (II) par extraction à l'aide d'un agent d'extraction (XI), avec obtention d'un mélange (XII) contenant de l'agent d'extraction (XI) et du lactame (II) et d'une phase (X), qui contient moins de lactame (II) que la phase (VI).

16. Procédé suivant la revendication 14 ou 15, dans lequel on recycle la phase (X) totalement ou partiellement dans l'étape a).

17. Procédé suivant les revendications 5 à 16, dans lequel, à partir du mélange (XII), on isole l'agent d'extraction (XI) et éventuellement des produits secondaires choisis parmi le groupe constitué des substances à faible point d'ébullition (VIII) et des substances à haut point d'ébullition (VII), avec obtention d'un lactame (II).

18. Procédé suivant les revendications 5 à 17, dans lequel on met en oeuvre le mélange (XII) et la phase (V) conjointement dans l'étape d).

19. Procédé suivant les revendications 5 à 18, dans lequel l'agent d'extraction (XI) et le diluant (III) sont constitués des mêmes composants ou présentent la même composition.

20. Procédé suivant les revendications 13 à 19, dans lequel on isole du lactame (II) uniquement à partir du courant partiel de la phase (VI) qui n'est pas recyclé dans l'étape de cyclisation et on recycle la partie restante de la phase (VI) totalement ou partiellement dans l'étape a), sans obtention de lactame (II).

21. Procédé suivant les revendications 1 à 20, dans lequel on recycle partiellement ou totalement dans l'étape a) du composé (I) de l'étape d) qui n'a pas réagi et a été isolé.

22. Procédé suivant les revendications 1 à 21, dans lequel on recycle totalement ou partiellement dans l'étape a) des substances à haut point d'ébullition (VII) isolées dans l'étape d).

23. Procédé suivant la revendication 22, dans lequel les substances à haut point d'ébullition (VII) isolées dans l'étape d) contiennent au moins 20 % en poids de lactame (II).

24. Procédé suivant les revendications 1 à 23, dans lequel on recycle partiellement ou totalement dans l'étape a) du diluant (III) isolé dans l'étape d).

25. Procédé suivant les revendications 1 à 24, dans lequel on recycle partiellement ou totalement dans l'étape a) des substances à faible point d'ébullition (VIII) isolées dans l'étape d).

26. Procédé suivant les revendications 15 à 25, dans lequel, comme agent d'extraction (XI), on met en oeuvre du diluant (III) isolé dans l'étape d).

27. Procédé suivant les revendications 1 à 26, dans lequel, à partir de la phase (V) ou de la phase (XII), on extrait à l'eau, totalement ou partiellement, du lactame (II) avec obtention d'une phase (XIII) appauvrie en lactame (II) et d'une phase (XIV) enrichie en lactame (II) et on recycle la phase (XIII) totalement ou partiellement dans l'étape a).

28. Procédé suivant les revendications 1 à 27, dans lequel, avant l'étape a), le courant de substances à haut point d'ébullition (VII) recyclé dans l'étape a) est réuni au courant recyclé (VI) ou (X).
